Europäisches Patentamt

European Patent Office

Office européen des brevets

(11) Publication number: 0 229 594
A2

# EUROPEAN PATENT APPLICATION

(21) Application number: 86810618.8

(22) Date of filing: 31.12.86

(51) Int. Cl.⁴: A23G 3/30 , A23G 3/00

(30) Priority: 07.01.86 US 816770

(43) Date of publication of application:
22.07.87 Bulletin 87/30

(84) Designated Contracting States:
DE ES FR GB GR NL SE

(71) Applicant: WARNER-LAMBERT COMPANY
201 Tabor Road
Morris Plains New Jersey 07950(US)

(72) Inventor: Huzinec, Robert J.
120 Kenvil Avenue
Kenvil New Jersey 07847(US)
Inventor: Graff, Allan H.
8 Blue Fern Lane
Randolph New Jersey 07869(US)

(74) Representative: Silbiger, Jakob, Dr.
c/o CAPSUGEL AG Münchensteinerstrasse
41
CH-4002 Basel(CH)

(54) Polyvinylprolidone-containing coating for comestibles.

(57) The invention provides a method for coating comestibles such as chewing gum, candy, tablets and pharmaceutical preparations using aqueous syrups containing polyvinylpyrrolidone and preferably a sweetener, which coatings are smooth, adherent and crunchy when chewed.

EP 0 229 594 A2

## POLYVINYLPYRROLIDONE-CONTAINING COATINGS FOR COMESTIBLES

This invention relates to sugar and sugarless coatings containing polyvinylpyrrolidone for comestibles such as chewing gums, confections or pharmaceutical preparations, tablets and the like. More particularly the invention is concerned with smooth and non-flaking sugar and sugarless coatings for comestibles made from coating syrups containing polyvinylpyrrolidone. These coatings are intended to be hard or crunchy in nature of the type commonly known in the pellet gums, e.g. CHICLET brand gum.

In the past, chewing gums in the form of shaped centers or cores, e.g. pellet gums, have been coated both with sugar and sugarless coatings to produce a candy-like, crunchy outer taste in the mouth with a chewable gum center portion. Conventionally, the core is a soft chewing gum portion, whereas the coating is crunchy or hard. In the case of sugarless coatings, aqueous solutions of xylitol, mannitol, maltitol and sorbitol, known as syrups, have been primarily employed. Such syrups may contain additives such as moisture absorbing compounds, anti-adherent compounds, dispersing agents, film forming agents binders and the like.

In U.S. Patent No. 4,307,838 a method for forming a sugarless coating on centers of chewing gum or other comestibles is described which includes the steps of applying to the centers a coating syrup which contains a sweetener such as sorbitol and/or other non-sugar sweetener, an adhesion or binder component such as gum arabic, a film forming component, an anti-adherent or filler component, and a dispersing agent to thereby coat the centers with the coating syrup, and optionally then applying a dusting mix to the centers coated with the coating syrup, the dusting mix including one or more sweeteners, such as employed in the coating syrup, in powdered form, and a moisture absorbing component, an anti-adherent component and a dispersing agent. See also UK Patent Application GB 2079129A which describes a similar method which further comprises applying a second coating syrup to smooth out the coating of the centers and provides a shine thereto, which second coating generally includes ingredients similar to those present in the dusting mix but dispersed in water.

One of the problems associated with forming coatings from these syrups is lumping and flaking of the finished coating rendering them unacceptable. The prior art has failed to disclose an effective coating or process to remedy this problem.

The present invention, on the other hand, employs an aqueous coating syrup for the use in coating a comestible shape containing polyvinylpyrrolidone and preferably sweeteners such as xylitol and sorbitol to provide uniform, smooth, and non-flaky coatings on comestible shapes, particularly chewing gum. The coatings must be resistant to enbrittlement, e.g., be flexible, yet have a hard crunchy bite.

In the past polyvinypyrrolidone has found application as an encapsulating agent for sweeteners and as a flavor enhancer. For example, in U.S. Patent No. 4,384,004 the preparation of the artificial sweetener L-aspartyl-L-phenylalanine methyl ester (APM) in encapsulated form is described in which the encapsulating materials include polyvinylpyrrolidone inter alia. The encapsulation can take place by a number of coating techniques including spray-drying, and coaservation, but preferably by the Wurster Process (similar to fluidized bed).

In U.S. Patent No. 4,48,789 a flavoring composition for chewing gum is described comprising a flavoring agent and a hydrophilic polymer such as polyvinylpyrrolidone.

Polyvinylpyrrolidone, however, has not been described heretofore as a syrup ingredient for forming sugarless coating on solid shapes of chewing gum or other comestibles.

In accordance with the invention an improved method is provided for forming a sugar or sugarless coating on a solid shape of chewing gum composition or other comestible resp. for preparing a coated shaped comestible which comprises applying to the shaped comestible a coating syrup comprising an aqueous solution of polyvinylpyrrolidone and preferably a sweetener and drying said syrup. The application of the coating syrup can be repeated, as many times as necessary to build up a desired coating weight and thickness on the shape.

For purposes of this invention, the term "comestible" is meant to include chewing gums, pharmaceutical preparations, confectionery products such as mints, lozenges, nougats, chocolate products and a wide variety of other solid or semi-solid edible products.

In carrying out the method of the invention, the coating syrup is formed as an aqueous solution of the polyvinylpyrrolidone, sweeteners and other additives, if desired.

The polyvinylpyrrolidone may be present in an amount from about 5% to about 60% by weight of the coating syrup and preferably from about 25% to about 55% by weight. The sweetener may be present in an amount of from about 30% to about 80% by weight of the coating syrup and preferably from about 40% to about 60% by weight with the remainder being water and minor amounts of optional additives, if employed.

The sweeteners suitable for use in the coating syrup comprise sugarless sweeteners such as the polyhydric alcohols, e.g., xylitol, sorbitol, mannitol, and mixtures, thereof, with xylitol and sorbitol being preferred; as well as maltitol, isomaltitol, hydrogenated starch hydrolysates, and hydrogenated glucose syrups. Mono, di-and polysaccharides may also be included. For example, sugars such as sucrose, fructose, glucose, galactose and maltose may also be employed as a sweetener. Other sweeteners suitable for use in the coating syrup include, but are not limited to free saccharin acid, water soluble salts of saccharin, cyclamate salts, palatinin, dihydrochalcones, glycyrrhizin, L-aspartyl-L-phenylalanine methyl ester, amino acid based sweeteners, talin, steviosides, dihydrochalcone compounds, acesulfame salts and mixtures thereof.

Other conventional coating components may be added in minor amounts to the coating syrup and include moisture absorbing compounds, anti-adherent compounds, binders, dispersing agents and film forming agents.

The moisture absorbing compounds suitable for use in the coating syrups include mannitol or dicalcium phosphate. Examples of useful anti-adherent compounds, which may also function as a filler, include talc, magnesium trisilicate and calcium carbonate. These ingredients may be employed in amounts of about 0.1% to about 5% by weight of the syrup and preferably 0.5 to about 2.0%.

Examples of binders include gum arabic, xanthan gum, gum tragacanth, topiocadextrin, or modified food starch with gum arabic being preferred.

Examples of dispersing agents which may be employed in the coating syrup include titanium dioxide, talc or other anti-adherent compounds as set forth above.

The coatings from the coating syrup are preferably applied by pan coating, although other conventional techniques such as by automated spray systems, fluidized bed granulation, may be useful. In pan coating methods, chewing gum pellet cores or other shapes are placed in a revolving coating pan and dedusted using cool dry air. The coating syrup is heated to about 50°C to 60°C and a portion thereof added to the revolving pan. Generally a single deposition of the coating syrup is not sufficient to provide the desired amount or thickness of coating deposited on the comestible. Accordingly, it usually will be necessary to apply second, third or more coats of the coating syrup in order to build up the weight and thickness of the coating to desired levels. However, before applying subsequent layers of the coating syrup, the previously applied layers are allowed to dry by gently flowing warm air or by adding a drying agent such as calcium carbonate or sorbitol in the case of sugarless coatings, or powdered sugar in the case of sugar coatings. Thereafter an additional portion of syrup is added followed by. drying with air or drying agent and this procedure is repeated until the desired coating weight is obtained. For example, in coating a chewing gum composition, the applications of coating syrup are continued until the average gum piece weight reaches the required coating weight. Thus, if the coating is to comprise about 35% by weight of the coated chewing gum tablet, application of 4 to 12 coats of coating syrup may be required. Other types of syrups may be added between portions of the inventive coating syrup such as an aqueous xylitol, maltitol or sorbitol solution in the case of sugarless coatings or a sugar solution in the case of sugar coatings. A flavorant such as peppermint oil, spearmint oil or the like may also be added. The distribution time, that is, the time during which the syrup of drying agent is mixed with the shapes is usually from 1 to 3 minutes. After the desired weight of coating has been obtained the coated shapes are dried with cool air (10-16°C).

In the case where the comestible to be coated is a chewing gum composition, flavoring agents and other conventional additives may be added to the gum composition. Such flavoring agents may comprise oils derived from plants, leaves, flowers, fruit etc. Representative flavor oils of this type include citrus oils such as lemon oil, orange oil, lime oil, grapefruit oil, fruit essences such as apple essence, pear essence, peach essence, strawberry essence, apricot essence, raspberry essence, cherry essence, plum essence, pineapple essence, as well as the following essential oils: peppermint oil, spearmint oil, mixtures of peppermint oil and spearmint oil, clove oil, bay oil, anise oil, eucalyptus oil, thyme oil, cedar leaf oil, cinnamon oil, oil of nutmeg, oil of sage, oil of bitter almonds, cassia oil, and methylsalicylate (oil of wintergreen). Various synthetic flavors such as mixed fruit, may also be incorporated.

Sweeteners suitable for use herein which may be present in the chewing gum composition center may comprise sugarless sweeteners such as the polyhydric alcohols,. e.g., xylitol, sorbitol, mannitol, and mixtures, thereof, with xylitol and sorbitol being preferred; as well as maltitol, isomaltitol, hydrogenated starch hydrolysates, and hydrogenated glucose syrups. Mono, di-and polysaccharides may also be

3

included. For example, sugars such as sucrose, fructose, glucose, galactose and maltose may also be employed as a sweetener. Other sweeteners suitable for use in the chewing gum composition centers include, but are not limited to free saccharin acid, water soluble salts of saccharin, cyclamate salts, palatinin, dihydrochalcones, glycyrrhizin, L-aspartyl-L-phenylalanine methyl ester, amino acid based sweeteners, talin, steviosides, dihydrochalcone compounds, acesulfame salts and mixtures thereof.

Where employed, natural sugars and/or natural sugar substitutes may be present in the chewing gum composition center. Such natural sweeteners suitable for use herein include polyhydric alcohols, such as sorbitol, xylitol, mannitol, isomaltitol, or maltitol. If desired, sugars such as mono-, di-and polysaccharides may also be employed. Representative examples include sucrose, dextrose, maltitose, fructose, maltodextrin, lactose and the like.

In general, the gum base is prepared by heating and blend various ingredients, such as natural gums, synthetic resin, waxes, plasticizers, etc, in a manner well known in the art. Typical examples of the ingredients found in a chewing gum base are masticatory substances of synthetic origin such as styrene-butadiene copolymer, isobutylene-isoprene copolymer, polyisobutylene, polyethylene, petroleum wax, polyvinyl acetate, as well as masticatory substances of natural origin such as rubber latex solids, chicle, crown gum, mispero, rosidinha, jelutong, pendare, perillo, niger gutta, tunu, etc. These elastomers will be employed in an amount within the range of about 5 to about 35%, preferably from about 15 to 30%, and optimally from about 18 to about 25% by weight of the total chewing gum composition.

The chewing gum composition may also include solvents, detackifiers, waxes, softening agents, lubricants, fillers, emulsifiers, colorants, antioxidants and/or texturizers, bulking agents and other conventional ingredients as will be apparent to those skilled in the art.

As indicated, in addition to chewing gum, the comestible to be coated may include any edible solid, such as candies, including hard candies and pressed candies, jelly beans, peanuts, other confections, as well as pharmaceutical preparations including pills, tablets or other solid dosage forms for medicinal or therapeutic use.

By employing the inventive syrup as the initial coating medium for comestibles such as gum, confectionery and pharmaceutical dosages forms, smooth and non-flaky coatings are produced.

In order to more completely describe the invention the following Examples are submitted, it being understood that such Examples are not intended to limit the scope of the invention. Percentages are by weight unless otherwise indicated.


Example 1

This example demonstrates the preparation of a sugarless coating on a sugarless chewing gum composition center according to the invention which coating is both uniform and adherent.

A sheet of gum centers or cores was prepared for all Examples herein from the ingredients set forth in Table 1 below.

## Table 1

| Ingredient | wt. % |
| --- | --- |
| Gum Base (Styrene Butadiene) | 27.21 |
| Sorbitol (granular) | 21.00 |
| Ingredient | wt. % |
| Glycerin | 5.23 |
| Sorbitol (solution) | 12.56 |
| Sorbitol (powder) | 32.40 |
| Flavorant (peppermint oil) | 1.60 |
| | 100.00 |

To prepare the centers, the gum base was melted and maintained at elevated temperatures. The glycerin was added followed by the solid sorbitols (granular and powder) which were added slowly with mixing. Thereafter the sorbitol solution was added followed by the flavor oil. The above mixture was mixed until homogeneous, cooled, rolled into sheets and scored to form a sheet of pillow shaped gum centers or cores.

A 10% polyvinylpyrrolidone coating syrup (Syrup #1) was prepared by combining 100 g of polyvinylpyrrolidone and 1000 g of $H_2O$.

The gum centers to be coated were placed in sheet form in a standard revolving coating pan and were broken up into individual centers. The centers were dedusted using cool dry air. Syrup #1 was heated to 50-52°C. The centers were coated four (4) times with 30 g portions of syrup #1 and dried with warm air after the last charge for 10 minutes.

The coatings were smooth, non-flaking and adherent. After 24 hours of conditioning at ambient temperature there was still no flaking of the coating.

Example 2-3

This Example demonstrates the coating of a sugarless gum with syrups containing polyvinylpyrrolidone and polyhydric alcohol sweeteners.

A second coating syrup (Coating Syrup #2) was prepared containing 50 g of polyvinylpyrrolidone, 780 g of xylitol and 220 g of water. A third coating syrup (Coating Syrup #3) was also prepared containing 50 g of polyvinyl pyrrolidone, 780 g of sorbitol and 220 g of water.

The sugarless gum centers were pan coated with syrup #2 in accordance with the scheme of Table 2 below.

Table 2 lists the charge, weight in grams of each charge, distribution time in minutes and the drying time in minutes.

<u>Table 2</u>

| Charge | Wt. (g) | Distribution Time (min) | Drying Time (min.) |
|---|---|---|---|
| 1 | 30 | 1 | – |
| 2 | 30 | 1 | – |
| 3 | 30 | 1 | – |
| 4 | 30 | 1 | 3 |
| 5 | 30 | 1 | – |
| 6 | 30 | 1 | – |
| 7 | 30 | 1 | – |
| 8 | 30 | 1 | 3 |
| 9 | 30 | 1 | – |
| 10 | 35 | 1 | – |
| 11 | 35 | 1 | 3 |
| 12 | 50 | 1 | – |
| 13 | 50 | 1 | – |
| 14 | 50 | 1 | 5 |
| 15 | 50 | 1 | – |
| 16 | 50 | 1 | – |
| 17 | 50 | 1 | – |
| 18 | 50 | 1/2 | 5 |
| 19 | 75 | 1 | – |
| 20 | 50 | 1 | – |
| 21 | 50 | 1 | – |
| 22 | 50 | 1/2 | 5 |

There was produced a 22.5% coating based on the weight of the gum centers. The coating was smooth, adherent, and after conditioning under ambient conditions for about 24 hours there was no signs of flaking.

The sugarless gum centers of Example 1 were then pan coated with Syrup #3 using the scheme of Table 3 below.

## Table 3

| Charge | Wt. (g) | Distribution Time (min) | Drying Time (min.) |
|--------|---------|-------------------------|--------------------|
| 1 | 30 | 1 | — |
| 2 | 30 | 1 | — |
| 3 | 30 | 1 | — |
| 4 | 30 | 1 | 7 |
| 5 | 30 | 1 | — |
| 6 | 30 | 1 | 7 |
| 7 | 30 | 1 | — |
| 8 | 30 | 1 | 7 |
| 9 | 30 | 1 | — |
| 10 | 35 | 1 | — |
| 11 | 35 | 1 | 7 |
| 12 | 50 | 1 | — |
| 13 | 50 | 1 | 7 |
| 14 | 50 | 1 | — |
| 15 | 50 | 1 | 7 |
| 16 | 50 | 1 | 5 |
| 17 | 50 | 1 | — |
| 18 | 50 | 1/2 | 5 |
| 19 | 75 | 1 | 7 |
| 20 | 50 | 1 | 5 |
| 21 | 50 | 1 | 5 |
| 22 | 50 | 1 | 10 |

There resulted a 27% coating by weight of the gum centers. The coating had good adherence but was slightly bumpy due to the moisture in the atmosphere. After conditioning the coating for 24 hours at ambient temperature there were no signs of flaking.

## Claims

1. A method for preparing a coated shaped comestible which comprises the steps of applying to said shaped comestible a coating syrup comprising an aqueous solution of polyvinylpyrrolidone and drying said syrup.

2. The method of claim 1 which further comprises a sweetener.

3. The method of claims 1 or 2 wherein said sweetener is a polyhydric alcohol selected from the group consisting of xylitol, sorbitol, mannitol, maltitol and mixtures thereof.

4. A method for preparing a sugarless coating on a comestible which comprises the steps of applying to said center a coating syrup comprising an aqueous solution of from about 5% to about 60% by weight of said syrup of polyvinylpyrrolidone and from about 30% to about 80% by weight of said syrup of a polyhydric alcohol selected from the group consisting of xylitol, sorbitol, mannitol, maltitol and mixtures thereof, and drying said syrup.

5. The shaped comestible coated according to any one of the claims 1 -4.

6. A coated confectionery or a pharmaceutical tablet according to claim 5.

7. A chewing gum product according to claim 5 having an inner core and a dry hard outer coating wherein the inner core comprises a chewing gum composition and the outer coating comprises polyvinylpyrrolidone and a sweetener.

8. An aqueous coating syrup for use in coating a comestible shape containing polyvinylpyrrolidone, as described in anyone of the claims 1 -4.